# EUROPEAN PATENT APPLICATION

(11) **EP 4 600 239 A1**
(43) Date of publication of application: **13.08.2025**
(21) Application number: 23874927.9
(22) Date of filing: 04.10.2023
(51) Int. Cl.: C07C 43/12, C11D 7/28, C09K 5/04

(54) **FLUORINE-CONTAINING ETHER COMPOUND AND METHOD FOR PRODUCING SAME**

(30) Priority: 04.10.2022 JP 2022160518
(71) Applicant: DAIKIN INDUSTRIES, LTD., Osaka-shi, Osaka 530-0001 (JP)
(72) Inventor: KISHIKAWA, Yosuke, Osaka-Shi, Osaka 530-0001 (JP); HOSHIYA, Naoyuki, Osaka-Shi, Osaka 530-0001 (JP); ISHIHARA, Sumi, Osaka-Shi, Osaka 530-0001 (JP)
(74) Representative: Hoffmann Eitle
(86) International application number: PCT/JP2023/036298
(87) International publication number: WO 2024/075802

(57) **Abstract**

Provided is a fluorine-containing ether compound represented by general formula: R¹-R²-O-R³-R⁴ wherein R¹ is - CH₃, -CH₂F, -CHF₂, -CH₂I, or -CHFI; R² is a fluorinated alkylene group having 1 to 10 carbon atoms consisting of unit represented by -CHF-, a fluorinated alkylene group having 2 to 10 carbon atoms consisting of unit represented by -CHF- and unit represented by -CH₂-, or a fluorine-containing alkylene group having 3 to 10 carbon atoms consisting of unit represented by -CFH-, unit represented by -CH₂-, and unit represented by -CHI-; R³ is single bond, a non-fluorinated alkylene group having 1 to 5 carbon atoms, or a fluorinated alkylene group having 1 to 10 carbon atoms; and R⁴ is -CH₃, - CH₂F, or -CHF₂.

## Description

### TECHNICAL FIELD

The present disclosure relates to a fluorine-containing ether compound and a method for producing the same.

### BACKGROUND ART

Fluorine-containing ethers are used not only as solvents, but also in a wide variety of applications.

As a fluorine-containing ether, for example, Patent Document 1 discloses a compound represented by general formula: Rh'(O-Rf)m wherein m = 3 to 4, Rf is independently a perfluoroaliphatic group, Rh' is independently a linear or branched hydrocarbon having 3 to about 8 carbon atoms.

For example, Patent Document 2 discloses a hydrofluoroether compound containing two terminal fluorinated alkyl groups and a substituted or unsubstituted oxymethylene group interposed in the chain, wherein each of the fluorinated alkyl groups contains only one hydrogen atom and optionally contains at least one heteroatom in the chain, provided that the hydrogen atom is a part of a monofluoromethylene moiety.

### RELATED ART

### PATENT DOCUMENTS

Patent Document 1: National Publication of International Patent Application No. 2008-529975
Patent Document 2: National Publication of International Patent Application No. 2009-507840

### SUMMARY OF THE INVENTION

### PROBLEM TO BE SOLVED BY THE INVENTION

As described above, Patent Documents 1 and 2 disclose that at least one group of two groups bonded to oxygen in the ether compound is formed of a perfluoroaliphatic group, or at least one group is formed of a fluorinated alkyl group containing only one hydrogen atom.

However, a fluorine-containing ether in which at least one group of two groups bonded to oxygen in the ether compound is formed of a group containing neither CF₃- (trifluoromethyl group) nor unit represented by -CF₂- (difluoromethylene group) has not been known.

An object of the present disclosure is to provide a fluorine-containing ether compound having a group containing neither trifluoromethyl group nor difluoromethylene group, as at least one group of two groups bonded to oxygen in the ether compound.

### MEANS FOR SOLVING THE PROBLEM

The present disclosure provides a fluorine-containing ether compound represented by general formula:

R¹-R²-O-R³-R⁴

wherein
R¹ is -CH₃, -CH₂F, -CHF₂, -CH₂I, or -CHFI;
R² is a fluorinated alkylene group having 1 to 10 carbon atoms consisting of unit represented by -CHF-, a fluorinated alkylene group having 2 to 10 carbon atoms consisting of unit represented by -CHF- and unit represented by -CH₂-, or a fluorine-containing alkylene group having 3 to 10 carbon atoms consisting of unit represented by -CFH-, unit represented by - CH₂-, and unit represented by -CHI-;
R³ is single bond, a non-fluorinated alkylene group having 1 to 5 carbon atoms, or a fluorinated alkylene group having 1 to 10 carbon atoms; and
R⁴ is -CH₃, -CH₂F, or -CHF₂.

### EFFECTS OF INVENTION

The present disclosure provides a fluorine-containing ether compound having a group containing neither trifluoromethyl group nor difluoromethylene group, as at least one group of two groups bonded to oxygen in the ether compound.

### DESCRIPTION OF EMBODIMENTS

Hereinafter, specific embodiments of the present disclosure will now be described in detail, but the present disclosure is not limited to the following embodiments.

The fluorine-containing ether compound of the present disclosure is a fluorine-containing ether compound represented by general formula:

R¹-R²-O-R³-R⁴

wherein R¹ is -CH₃, -CH₂F, -CHF₂, -CH₂I, or -CHFI; R² is a fluorinated alkylene group having 1 to 10 carbon atoms consisting of a unit represented by -CHF-, a fluorinated alkylene group having 2 to 10 carbon atoms consisting of a unit represented by -CHF- and a unit represented by -CH₂-, or a fluorine-containing alkylene group having 3 to 10 carbon atoms consisting of a unit represented by -CFH-, a unit represented by -CH₂-, and a unit represented by -CHI-; R³ is a single bond, a non-fluorinated alkylene group having 1 to 5 carbon atoms, or a fluorinated alkylene group having 1 to 10 carbon atoms; and R⁴ is -CH₃, -CH₂F, or -CHF₂.

R¹ is -CH₃, -CH₂F, -CHF₂, -CH₂I, or -CHFI, preferably - CH₂F, -CHF₂, or -CHFI, and more preferably -CH₂F. The fluorine-containing ether compound of the present disclosure has a feature that at least one group of two groups bonded to oxygen in the ether compound has no CF₃- (trifluoromethyl group) at the terminal.

R² is a fluorinated alkylene group having 1 to 10 carbon atoms consisting of a unit represented by -CHF-, a fluorinated alkylene group having 2 to 10 carbon atoms consisting of a unit represented by -CHF- and a unit represented by -CH₂-, or a fluorine-containing alkylene group having 3 to 10 carbon atoms consisting of a unit represented by -CFH-, a unit represented by -CH₂-, and a unit represented by -CHI-. The fluorine-containing ether compound of the present disclosure has a feature that at least one group of two groups bonded to oxygen in the ether compound certainly contains a unit represented by -CHF-, and contains no unit represented by -CF₂-.

When R² is a fluorinated alkylene group consisting of a unit represented by -CHF-, R² is preferably, for example, a fluorinated alkylene group represented by -(CHF)ₙ₁- wherein n1 is an integer of 1 to 10.

When R² is a fluorinated alkylene group consisting of a unit represented by -CHF- and a unit represented by -CH₂-, examples of R² include a fluorinated alkylene group represented by -CHF-(CHF-CHF)ₙ₂-(CH₂)ₘ₁- wherein n2 is an integer of 1 to 4 and m1 is an integer of 1 or more, and a fluorinated alkylene group represented by -(CHF)ₙ₇-CH₂-CH₂-(CH₂)_{q}- wherein n7 is an integer of 1 to 7 and q is an integer of 1 to 6.

When R² is a fluorinated alkylene group consisting of a unit represented by -CHF- and a unit represented by -CH₂-, R² is preferably a fluorinated alkylene group represented by - (CHF)ₙ₂-CH₂- wherein n2 is an integer of 1 to 9, or a fluorinated alkylene group represented by -(CHF)ₙ₇-CH₂-CH₂-(CH₂)_{q}- wherein n7 is an integer of 1 to 7 and q is an integer of 1 to 6, and more preferably a fluorinated alkylene group represented by -CHF-(CHF-CHF)ₙ₃-CH₂- wherein n3 is an integer of 0 to 4, or -CHF-(CHF-CHF)ₙ₈-CH₂-CH₂-(CH₂)_{q}- wherein n8 is an integer of 0 to 2 and q is an integer of 1 to 6.

When R² is an alkylene group consisting of a unit represented by -CFH-, a unit represented by -CH₂-, and a unit represented by -CHI-, R² is preferably a fluorinated alkylene group represented by -(CHF)ₙ₇-CH₂-CHI-(CH₂)_{q}- wherein n7 is an integer of 1 to 7 and q is an integer of 1 to 6, and more preferably a fluorinated alkylene group represented by -CHF-(CHF-CHF)ₙ₈-CH₂-CHI-(CH₂)_{q}- wherein n8 is an integer of 0 to 2 and q is an integer of 1 to 6.

In the general formula representing the fluorine-containing ether compound, the group represented by R¹-R²- is preferably CH₂F-CHF- or a fluorinated alkyl group represented by general formula: CH₂F-CHF-(CHF-CHF)ₙ₃-CH₂- wherein n3 is an integer of 0 to 4, or a fluorinated alkyl group represented by general formula: CHF₂-CHF- (CHF-CHF)ₙ₈-CH₂-CHX¹¹-(CH₂)_{q}- wherein n8 is an integer of 0 to 2, X¹¹ is I or H, and q is an integer of 1 to 6.

n3 is preferably 0 or 1.

R³ is a single bond, a non-fluorinated alkylene group having 1 to 5 carbon atoms or a fluorinated alkylene group having 1 to 10 carbon atoms. The number of carbon atoms of R³ is preferably 0 to 5, more preferably 0 to 3, and further preferably 0 or 1. The number of carbon atoms of the non-fluorinated alkylene group in R³ is preferably 1 or 2. The number of carbon atoms of the fluorinated alkylene group in R³ is preferably 1 to 5, and more preferably 1 or 2.

R³ is preferably a single bond, a non-fluorinated alkylene group having 1 to 5 carbon atoms, a fluorinated alkylene group represented by -(CHF)ₙ₄- wherein n4 is an integer of 1 to 10, or a fluorinated alkylene group represented by -(CHF)ₙ₅-CH₂- wherein n5 is an integer of 1 to 9.

R⁴ is -CH₃, -CH₂F, or -CHF₂, and is preferably -CH₃ or - CH₂F.

In the general formula representing the fluorine-containing ether compound, the group represented by R⁴-R³- is preferably a non-fluorinated alkyl group having 1 to 4 carbon atoms, or a fluorinated alkyl group represented by general formula:

CH₂F-CHF-(CHF-CHF)ₙ₆-CH₂-

wherein n6 is an integer of 0 to 4.

When the group represented by R⁴-R³- is a non-fluorinated alkyl group, the number of carbon atoms of the non-fluorinated alkyl group is preferably 1 or 2.

n6 is preferably 0 or 1.

The fluorine-containing ether compound is preferably at least one selected from the group consisting of CH₂FCHFOCH₃, CH₂FCHFCH₂OCH₃, CH₂FCHFCH₂OCHFCFH₂, CH₂FCHFCH₂OCH₂CH₃, CH₂FCHFCHFCHFCH₂OCH₃, CHF₂-CHF-CH₂-CHI-CH₂-OCH₃, CHF₂-CHF-CH₂-CH₂-CH₂-OCH₃, CHF₂-CHF-CH₂-CH₂-CH₂-OCHFCFH₂, CHF₂-CHF-CH₂-CH₂-CH₂-OCH₂CH₃, CHF₂-CHF-CH₂-CHI-CH₂-CH₂-CH₂-CH₂-OCH₃, and CHF₂-CHF-CH₂-CH₂-CH₂-CH₂-CH₂-CH₂-OCH₃.

The fluorine-containing ether compound of the present disclosure can be produced by, for example, a production method in which CHF=CHF is reacted with an alcohol represented by general formula: R⁴-R³-OH wherein R³ and R⁴ are as described above in the presence of a basic compound to produce a fluorine-containing ether compound represented by general formula: CH₂F-CHF-O-R³-R⁴ wherein R³ and R⁴ are as described above (hereinafter, sometimes referred to as the first production method).

In the first production method, the basic compound acts as a catalyst. The basic compound is preferably an inorganic basic compound, and is more preferably an alkali metal hydroxide or an alkaline earth metal hydroxide, such as NaOH, KOH, CsOH, LiOH, Ca(OH)₂, or Ba(OH)₂.

The amount of the basic compound used is preferably 0.01 to 1.0 mol, and more preferably 0.2 to 0.8 mol per 1 mol of the alcohol.

The reaction of CHF=CHF with the alcohol can be performed in a solvent. Examples of the solvent include polar organic solvents such as water, diethyl ether, glyme, dioxane, tetrahydrofuran, and acetonitrile.

The reaction pressure of CHF=CHF with the alcohol is preferably 1.0 to 2.0 MPaG. The reaction temperature of CHF=CHF with the alcohol is preferably 20 to 95°C.

The fluorine-containing ether compound of the present disclosure can be produced by, for example, a production method in which
CHF=CHF is added to methanol to produce a fluorine-containing alcohol represented by general formula: CH₂F-CHF-(CHF-CHF)ₙ₃-CH₂OH wherein n3 is an integer of 0 to 4, and
the fluorine-containing alcohol is reacted with a compound represented by R⁴-R³-X wherein R³ and R⁴ are as described above, X is a halogen atom or -OSO₃R' (wherein R' is a non-fluorinated alkyl group or a fluorinated alkyl group) to produce a fluorine-containing ether compound represented by general formula: CH₂F-CHF-(CHF-CHF)ₙ₃-CH₂-O-R³-R⁴ wherein n3, R³, and R⁴ are as described above (hereinafter, sometimes referred to as the second production method).

In the second production method, first, the fluorine-containing alcohol is produced by adding CHF=CHF to methanol.

n3 in the fluorine-containing alcohol represents the degree of polymerization of CHF=CHF. n3 is an integer of 0 to 4, and preferably 0 or 1.

The reaction of methanol with CHF=CHF can be performed in the presence of a radical initiator. When the reaction is performed in the presence of a radical initiator, the radical initiator is decomposed to generate radicals, a generated radical draws out a hydrogen atom on the carbon atom to which the hydroxyl group of methanol is bonded, to generate a methanol radical, and a reaction of adding CHF=CHF to the methanol radical (a so-called telomerization reaction) proceeds.

The radical initiator is preferably an organic peroxide, and preferable examples thereof include dialkyl peroxycarbonates such as diisopropyl peroxydicarbonate and di sec-butyl peroxydicarbonate; peroxyesters such as 2-ethylhexanoyl (tert-butyl) peroxide, t-butyl peroxyisobutyrate, and t-butyl peroxypivalate; and dialkyl peroxides such as di t-butyl peroxide. Among these, the radical initiator is preferably di t-butyl peroxide.

The amount of CHF=CHF used is preferably 0.01 to 100 mol per 1 mol of the methanol.

The amount of the radical initiator used is preferably 0.01 to 2 mol per 1 mol of the methanol.

The reaction temperature of the methanol with CHF=CHF can be suitably selected, and is preferably -78 to 200°C. The reaction temperature of the methanol with CHF=CHF is preferably the decomposition temperature of the radical polymerization initiator or more, and preferably less than the decomposition temperatures of the substrate and the product.

The reaction pressure of the methanol with CHF=CHF can be suitably selected, and is preferably 0 to 5.0 MPaG. The reaction time of the methanol with CHF=CHF can be suitably selected, and is preferably 0.1 to 96 hours.

In the second production method, then, the obtained fluorine-containing alcohol is reacted with the compound represented by R⁴-R³-X to alkylate oxygen in the fluorine-containing alcohol, thereby producing the fluorine-containing ether compound.

When X is a halogen atom, the fluorine-containing ether compound can be produced by acting a base such as sodium hydride on the fluorine-containing alcohol, and reacting the resulting fluorine-containing alkoxide with the compound represented by R⁴-R³-X wherein X is a halogen atom.

When X is -OSO₃R' wherein R' is a non-fluorinated alkyl group or a fluorinated alkyl group, for example, the fluorine-containing ether compound can be produced by reacting the fluorine-containing alcohol with a compound represented by R⁴-R³-X wherein X is -OSO₃R' in an aqueous solution of a hydroxide.

The compound represented by R⁴-R³-X wherein X is -OSO₃R' is preferably (R⁴-R³-O-)₂SO₂ wherein R³ and R⁴ are as described above.

The reaction pressure of the fluorine-containing alcohol with the compound represented by R⁴-R³-X is preferably 0.1 to 2.0 MPaG. The reaction temperature of the fluorine-containing alcohol with the compound represented by R⁴-R³-X is preferably 20 to 95°C.

The fluorine-containing ether compound of the present disclosure can be produced by, for example, a production method in which
CHF=CHF is reacted with I₂ and IF₅ to produce a first fluorine-containing alkyliodide represented by general formula: R¹-CHF-I wherein R¹ is -CHF₂ or -CHFI, CHF=CHF is added to the first fluorine-containing alkyliodide to produce a second fluorine-containing alkyliodide represented by general formula: R¹-CHF-(CHF-CHF)ₙ₈-I wherein R¹ is as described above and n8 is 1 or 2,
the first or second fluorine-containing alkyliodide is reacted with an unsaturated compound represented by general formula: CH₂=CH-(CH₂)_{q}-OH wherein q is an integer of 1 or more to produce a first fluorine-containing alcohol represented by general formula: R¹-CHF-(CHF-CHF)ₙ₈-CH₂-CHI-(CH₂)_{q}-OH wherein R¹ is as described above, n8 is an integer of 0 to 2, and q is an integer of 1 to 6,
the first fluorine-containing alcohol is optionally reduced to produce a first fluorine-containing alcohol represented by general formula: R¹-CHF-(CHF-CHF)ₙ₈-CH₂-CH₂-(CH₂)_{q}-OH wherein R¹ is as described above, n8 is an integer of 0 to 2, and q is an integer of 1 to 6, and the first or second fluorine-containing alcohol is reacted with a compound represented by R⁴-R³-X wherein R³ and R⁴ are as described above, X is a halogen atom or -OSO₃R' wherein R' is a non-fluorinated alkyl group or a fluorinated alkyl group to produce a fluorine-containing ether compound represented by general formula: R¹-CHF-(CHF-CHF)ₙ₈-CH₂-CHX¹¹-(CH₂)_{q}-O-R³-R⁴ wherein n8 is an integer of 0 to 2, X¹¹ is I or H, q is an integer of 1 to 6, and R³ and R⁴ are as described above (hereinafter, sometimes referred to as the third production method).

In the third production method, first, CHF=CHF is reacted with I₂ and IF₅ to produce the first fluorine-containing alkyliodide represented by the general formula: R¹-CHF-I wherein R¹ is -CHF₂ or -CHFI, CHF=CHF is added to the first fluorine-containing alkyliodide to produce the second fluorine-containing alkyliodide represented by the general formula: R¹-CHF-(CHF-CHF)ₙ₈-I wherein R¹ is as described above and n8 is 1 or 2.

The amount of I₂ and IF₅ used is preferably 0.5 to 2 mol per 1 mol of CHF=CHF.

The reaction of CHF=CHF with I₂ and IF₅ can be performed in a solvent.

The reaction temperature of CHF=CHF with I₂ and IF₅ can be suitably selected, and is preferably -78 to 200°C. The reaction pressure of CHF=CHF with I₂ and IF₅ can be suitably selected, and is preferably 0 to 5.0 MPaG. The reaction time of CHF=CHF with I₂ and IF₅ can be suitably selected, and is preferably 0.1 to 96 hours.

The reaction of the first fluorine-containing alkyl iodide with CHF=CHF is a telomerization reaction in which the first fluorine-containing alkyl iodide is a telogen and CHF=CHF is a taxogen, and this reaction produces the second fluorine-containing alkyl iodide.

n8 in the second fluorine-containing alkyl iodide represents the degree of polymerization of CHF=CHF. n8 is 1 or 2, preferably 1.

The reaction of the first fluorine-containing alkyl iodide with CHF=CHF can be performed in the presence of a radical initiator. Examples of the radical initiator include organic peroxides and azo compounds.

Examples of the organic peroxide include dialkyl peroxycarbonates such as diisopropyl peroxydicarbonate and di sec-butyl peroxydicarbonate; peroxyesters such as 2-ethylhexanoyl (tert-butyl) peroxide, t-butyl peroxyisobutyrate, and t-butyl peroxypivalate; and dialkyl peroxides such as di t-butyl peroxide.

Examples of the azo compound include azobisisobutyronitrile.

The amount of CHF=CHF used is preferably 0.01 to 100 mol per 1 mol of the fluorine-containing alkyl iodide.

The amount of the radical initiator used is preferably 0.01 to 2 mol per 1 mol of the fluorine-containing alkyl iodide.

The reaction temperature of the first fluorine-containing alkyl iodide with CHF=CHF can be suitably selected, and is preferably -78 to 200°C. The reaction temperature of the first fluorine-containing alkyl iodide with CHF=CHF is preferably the decomposition temperature of the radical polymerization initiator or more, and is preferably less than the decomposition temperatures of the substrate and the product.

The reaction pressure of the first fluorine-containing alkyl iodide with CHF=CHF can be suitably selected, and is preferably 0 to 5.0 MPaG. The reaction time of the first fluorine-containing alkyl iodide with CHF=CHF can be suitably selected, and is preferably 0.1 to 96 hours.

In the third production method, then, the first or second fluorine-containing alkyliodide is reacted with the unsaturated compound represented by the general formula: CH₂=CH-(CH₂)_{q}-OH wherein q is an integer of 1 or more to produce the fluorine-containing alcohol represented by the general formula: R¹-CHF-(CHF-CHF)ₙ₈-CH₂-CHI-(CH₂)_{q}-OH wherein R¹ is as described above, n8 is an integer of 0 to 2, and q is an integer of 1 to 6.

R¹ in the fluorine-containing alcohol is the same as R¹ in the first or second fluorine-containing alkyliodide, and is -CHF₂ or -CHFI.

n8 in the fluorine-containing alcohol is an integer of 0 to 2, and is preferably 0 or 1.

The reaction of the first or second fluorine-containing alkyl iodide with the unsaturated compound can be performed in the presence of a compound generating radicals. Examples of such a compound include organic peroxides and azo compounds.

Examples of the organic peroxide include dialkyl peroxycarbonates such as diisopropyl peroxydicarbonate and di sec-butyl peroxydicarbonate; peroxyesters such as 2-ethylhexanoyl (tert-butyl) peroxide, t-butyl peroxyisobutyrate, and t-butyl peroxypivalate; and dialkyl peroxides such as di t-butyl peroxide.

Examples of the azo compound include azobisisobutyronitrile.

The amount of the unsaturated compound used is preferably 0.01 to 100 mol per 1 mol of the first or second fluorine-containing alkyl iodide.

The amount of the compound generating radicals used is preferably 0.001 to 1 mol per 1 mol of the first or second fluorine-containing alkyl iodide.

The reaction temperature of the first or second fluorine-containing alkyl iodide with unsaturated compound can be suitably selected, and is preferably 50 to 200°C. The reaction pressure of the first or second fluorine-containing alkyl iodide with unsaturated compound can be suitably selected, and is preferably 0.1 to 5 MPaG. The reaction time of the first or second fluorine-containing alkyl iodide with unsaturated compound can be suitably selected, and is preferably 0.1 to 96 hours.

In the third production method, then, the obtained fluorine-containing alcohol is reacted with the compound represented by R⁴-R³-X to alkylate oxygen in the fluorine-containing alcohol, thereby producing the fluorine-containing ether compound.

When X is a halogen atom, the fluorine-containing ether compound can be produced by acting a base such as sodium hydride on the fluorine-containing alcohol, and reacting the resulting fluorine-containing alkoxide with the compound represented by R⁴-R³-X wherein X is a halogen atom.

When X is -OSO₃R' wherein R' is a non-fluorinated alkyl group or a fluorinated alkyl group, for example, the fluorine-containing ether compound can be produced by reacting the fluorine-containing alcohol with a compound represented by R⁴-R³-X wherein X is -OSO₃R' in an aqueous solution of a hydroxide.

The compound represented by R⁴-R³-X wherein X is -OSO₃R' is preferably (R⁴-R³-O-)₂SO₂ wherein R³ and R⁴ are as described above.

The reaction pressure of the fluorine-containing alcohol with the compound represented by R⁴-R³-X is preferably 0.1 to 2.0 MPaG. The reaction temperature of the fluorine-containing alcohol with the compound represented by R⁴-R³-X is preferably 20 to 95°C.

In the third production method, the first fluorine-containing alcohol may be reduced to the second fluorine-containing alcohol. The reduction can be performed by, for example, using a metal catalyst and hydrogen, or using zinc as a reducing agent.

The fluorine-containing ether compound of the present disclosure can be produced by, for example, a production method in which
CHF=CHF is reacted with I₂ and IF₅ to produce a first fluorine-containing alkyliodide represented by general formula: R¹-CHF-I wherein R¹ is -CHF₂ or -CHFI, CHF=CHF is added to the first fluorine-containing alkyliodide to produce a second fluorine-containing alkyliodide represented by general formula: R¹-CHF-(CHF-CHF)ₙ₈-I wherein R¹ is as described above and n8 is 1 or 2,
the first or second fluorine-containing alkyliodide is reacted with an unsaturated compound represented by general formula: CH₂=CH-(CH₂)_{q}-O-R³-R⁴ wherein q is an integer of 1 or more, R³ and R⁴ are as described as R³ and R⁴ contained in the fluorine-containing ether compound of the present disclosure to produce a fluorine-containing ether compound represented by general formula: R¹-CHF-(CHF-CHF)ₙ₈-CH₂-CHI-(CH₂)_{q}-O-R³-R⁴ wherein R¹, R³, and R⁴ are as described above, n8 is an integer of 0 to 2, and q is an integer of 1 to 6 (hereinafter, sometimes referred to as the fourth production method).

The production methods of the first and second fluorine-containing alkyliodides are as described in the third production method.

The reaction of the first or second fluorine-containing alkyliodide with the unsaturated compound represented by the general formula: CH₂=CH-(CH₂)_{q}-O-R³-R⁴ can be performed in the same manner as the method described in the third production method as the reaction of the first or second fluorine-containing alkyliodide with the unsaturated compound represented by the general formula: CH₂=CH-(CH₂)_{q}-OH.

The obtained fluorine-containing ether compound may be reduced to produce a fluorine-containing ether compound represented by the general formula: R¹-CHF-(CHF-CHF)ₙ₈-CH₂-CH₂-(CH₂)_{q}-O-R³-R⁴ wherein R¹, R³, and R⁴ are as described above, and n8 is an integer of 0 to 2 and q is an integer of 1 to 6. The reduction can be performed by, for example, using a metal catalyst and hydrogen, or using zinc as a reducing agent.

The composition of the present disclosure may contain the above fluorine-containing ether compound and a solvent. For example, the solvent may be at least one selected from the group consisting of an alcohol, an ether, an alkane, an alkene, a perfluorinated carbon, a perfluorinated tertiary amine, a perfluorinated ether, a cycloalkane, an ester, a ketone, an aromatic compound, a siloxane, a hydrochlorocarbon, a hydrochlofluorocarbon, and a hydrofluorocarbon.

The composition of the present disclosure may contain the above fluorine-containing ether compound, and a further component such as a surfactant, a stabilizer, a pigment, a dye, a colorant, an antioxidant, or a flame retarder.

The composition of the present disclosure may contain the above fluorine-containing ether compound, and a further fluorine-containing ether compound that is different from the above fluorine-containing ether compound. Examples of the further fluorine-containing ether compound include a hydrofluoroether compound.

The fluorine-containing ether compound of the present disclosure and the composition of the present disclosure can be suitably used as a heat-transfer fluid, a solvent for cleaning, a bubble size adjuster for adjusting the bubble size of a foamed heat-insulating material, a fire extinguishing agent, a carrier fluid, a working fluid, a solvent for polymerization, an abrasive, a desiccant, a resist developer, a resist stripper, or the like. For example, the fluorine-containing ether compound of the present disclosure and the composition of the present disclosure can be used in applications of the hydrofluoroether compound described in Japanese National Publication of International Patent Application No. 2009/507840.

While embodiments have been described above, it will be understood that various changes in form and detail can be made without departing from the gist and scope of the claims.

The main embodiments of the present disclosure are as follows.
<1> According to the first aspect of the present disclosure, provided is a fluorine-containing ether compound represented by general formula:

   R¹-R²-O-R³-R⁴

   wherein
   R¹ is -CH₃, -CH₂F, -CHF₂, -CH₂I, or -CHFI;
   R² is a fluorinated alkylene group having 1 to 10 carbon atoms consisting of unit represented by -CHF-, a fluorinated alkylene group having 2 to 10 carbon atoms consisting of unit represented by -CHF- and unit represented by -CH₂-, or a fluorine-containing alkylene group having 3 to 10 carbon atoms consisting of unit represented by -CFH-, unit represented by - CH₂-, and unit represented by -CHI-;
   R³ is single bond, a non-fluorinated alkylene group having 1 to 5 carbon atoms, or a fluorinated alkylene group having 1 to 10 carbon atoms; and
   R⁴ is -CH₃, -CH₂F, or -CHF₂.
<2> According to the second aspect of the present disclosure, provided is the fluorine-containing ether compound according to the first aspect, wherein R¹ is -CH₂F, -CHF₂, or -CHFI.
<3> According to the third aspect of the present disclosure, provided is the fluorine-containing ether compound according to the first or second aspect, wherein
   R² is a fluorinated alkylene group represented by general formula:

      -(CHF)ₙ₁-

      wherein n1 is an integer of 1 to 10;
   a fluorinated alkylene group represented by general formula:

      -(CHF)ₙ₂-CH₂-

      wherein n2 is an integer of 1 to 9;
   a fluorinated alkylene group represented by:

      -(CHF)ₙ₇-CH₂-CH₂-(CH₂)_{q}-

      wherein n7 is an integer of 1 to 7, and q is an integer of 1 to 6; or
   a fluorinated alkylene group represented by:

      -(CHF)ₙ₇-CH₂-CHI-(CH₂)_{q}-

      wherein n7 is an integer of 1 to 7, and q is an integer of 1 to 6.
<4> According to the fourth aspect of the present disclosure, provided is the fluorine-containing ether compound according to any one of the first to third aspects, wherein

   R¹-R²-

   is

   CH₂F-CHF-;

   a fluorinated alkyl group represented by general formula:

      CH₂F-CHF-(CHF-CHF)ₙ₃-CH₂-

      wherein n3 is an integer of 0 to 4; or
   a fluorinated alkyl group represented by general formula:

      CHF₂-CHF-(CHF-CHF)ₙ₈-CH₂-CHX¹¹-(CH₂)_{q}-

      wherein n8 is an integer of 0 to 2, X¹¹ is I or H, and q is an integer of 1 to 6.
<5> According to the fifth aspect of the present disclosure, provided is the fluorine-containing ether compound according to any one of the first to fourth aspects, wherein
   R³ is a single bond; a non-fluorinated alkylene group having 1 to 5 carbon atoms;
   a fluorinated alkylene group represented by general formula:

      -(CHF)ₙ₄-

      wherein n4 is an integer of 1 to 10; or
   a fluorinated alkylene group represented by general formula:

      -(CHF)ₙ₅-CH₂-

      wherein n5 is an integer of 1 to 9.
<6> According to the sixth aspect of the present disclosure, provided is the fluorine-containing ether compound according to any one of the first to fifth aspects, wherein

   R⁴-R³-

   is
   a non-fluorinated alkyl group having 1 to 4 carbon atoms; or
   a fluorinated alkyl group represented by general formula:

      CH₂F-CHF-(CHF-CHF)ₙ₆-CH₂-

      wherein n6 is an integer of 0 to 4.
<7> According to the seventh aspect of the present disclosure, provided is the fluorine-containing ether compound according to any one of the first to sixth aspects, wherein the fluorine-containing ether compound is at least one selected from the group consisting of CH₂FCHFOCH₃, CH₂FCHFCH₂OCH₃, CH₂FCHFCH₂OCHFCFH₂, CH₂FCHFCH₂OCH₂CH₃, CH₂FCHFCHFCHFCH₂OCH₃, CHF₂-CHF-CH₂-CHI-CH₂-OCH₃, CHF₂-CHF-CH₂-CH₂-CH₂-OCH₃, CHF₂-CHF-CH₂-CH₂-CH₂-OCHFCFH₂, CHF₂-CHF-CH₂-CH₂-CH₂-OCH₂CH₃, CHF₂-CHF-CH₂-CHI-CH₂-CH₂-CH₂-CH₂-OCH₃, and CHF₂-CHF-CH₂-CH₂-CH₂-CH₂-CH₂-CH₂-OCH₃.
<8> According to the eighth aspect of the present disclosure, provided is a composition comprising the fluorine-containing ether compound according to any one of the first to seventh aspects, and at least one selected from the group consisting of an alcohol, an ether, an alkane, an alkene, a perfluorinated carbon, a perfluorinated tertiary amine, a perfluorinated ether, a cycloalkane, an ester, a ketone, an aromatic compound, a siloxane, a hydrochlorocarbon, a hydrochlofluorocarbon, and a hydrofluorocarbon.
<9> According to the ninth aspect of the present disclosure, provided is a heat-transfer fluid comprising the fluorine-containing ether compound according to any one of the first to seventh aspects, or the composition according to the eighth aspect.
<10> According to the tenth aspect of the present disclosure, provided is a solvent for cleaning comprising the fluorine-containing ether compound according to any one of the first to seventh aspects, or the composition according to the eighth aspect.

### EXAMPLES

Next, embodiments of the present disclosure will now be described by way of Examples, but the present disclosure is not limited only to the Examples.

### Example 1 Synthesis of 1,1,2-trifluoro-2-iodoethane

To a 300 mL pressure resistant vessel, 37.1 g of iodine and 16.1 g of IF₅ were added, and the container was sealed. The container was cooled to -78°C, 10 g of (E)-1,2-difluoroethene was then introduced into the container, and the container was heated at 80°C for 20 hours. After the container was cooled with ice water, the content in the pressure resistant vessel was washed with water and then further washed with a 5% aqueous Na₂S₂O₄ solution to give 5.8 g of the title compound.
¹⁹F NMR (282 MHz, CDCl₃): δ -169.1 - -169.4 (m, 1F), -124.0 - - 124.3 (m, 1F).
¹H NMR (400 MHz, CDCl3): δ 6.79 (d with fine coupling, J = 48.0 Hz, 1H), 7.26 (td with fine coupling, J = 54.8, 3.6 Hz, 1H). LRMS (EI 70 eV) m/z (%): 210 (M+, 100), 190 (8), 171 (3), 83 (62), 64 (37), 51 (14).

### Example 2 Synthesis of 4,5,5-trifluoro-2-iodopentanol

To a 10 mL pressure resistant vessel, 1.84 g of 1,1,2-trifluoro-2-iodoethane, 509 mg of allyl alcohol, and 288 mg of azobisisobutyronitrile were added. Then, the container was heated at 80°C for 22 hours. After the container was cooled with ice water, the content in the pressure resistant vessel was analyzed by gas chromatography mass spectrometry, and it was found that the title compound was produced in an area ratio of 75.9% relative to an area ratio of 24.1% of 1,1,2-trifluoro-2-iodoethane as the raw material.
LRMS (EI 70 eV) m/z (%): 268 (M+, 1), 251 (1), 185 (2), 141 (95), 73 (100), 51 (38).

### Example 3 Synthesis of 1,1,2-trifluoro-4-iodo-8-methoxyoctane

To a 10 mL pressure resistant vessel, 500 mg of 1,1,2-trifluoro-2-iodoethane, 272 mg of 6-methoxyhexene, and 117 mg of azobisisobutyronitrile were added. The vessel was cooled to -78°C and then purged with nitrogen, and nitrogen was then added to 0.5 MPa. The vessel was heated at 80°C for 13 hours. After the vessel was cooled with ice water, a mixture containing the target compound was obtained. The content was analyzed by gas chromatography mass spectrometry, and it was found that the title compound was produced in an area ratio of 65.8% (two isomers in total) relative to an area ratio of 34.2% of 1,1,2-trifluoro-2-iodoethane as the raw material. LRMS (EI 70 eV) m/z (%): 197 ([M-I]⁺, 37), 165 (100), 45 (50).

### Example 4 Synthesis of 1,1,2-trifluoro-8-methoxyoctane

To a 10 mL glass container, 200 mg of 1,1,2-trifluoro-4-iodo-8-methoxyoctane, 0.4 mL of methanol, and 80.7 mg of Zn were added. Four drops of a 2 M aqueous HCl solution were added, the container was then cooled to -78°C and purged with nitrogen, and then stirred at room temperature for 2 hours. The content in the container was analyzed by gas chromatography mass spectrometry, and it was found that the raw materials were lost and the title compound was produced. LRMS (EI 70 eV) m/z (%): 166 ([M-CH₃OH]⁺, 11), 138 (27), 51 (9), 45 (100).

## Claims

1. A fluorine-containing ether compound represented by general formula:
R¹-R²-O-R³-R⁴
wherein
R¹ is -CH₃, -CH₂F, -CHF₂, -CH₂I, or -CHFI;
R² is a fluorinated alkylene group having 1 to 10 carbon atoms consisting of unit represented by -CHF-, a fluorinated alkylene group having 2 to 10 carbon atoms consisting of unit represented by -CHF- and unit represented by -CH₂-, or a fluorine-containing alkylene group having 3 to 10 carbon atoms consisting of unit represented by -CFH-, unit represented by - CH₂-, and unit represented by -CHI-;
R³ is single bond, a non-fluorinated alkylene group having 1 to 5 carbon atoms, or a fluorinated alkylene group having 1 to 10 carbon atoms; and
R⁴ is -CH₃, -CH₂F, or -CHF₂.

2. The fluorine-containing ether compound according to claim 1, wherein R¹ is -CH₂F, -CHF₂, or -CHFI.

3. The fluorine-containing ether compound according to claim 1 or 2, wherein
R² is a fluorinated alkylene group represented by general formula:
-(CHF)ₙ₁-
wherein n1 is an integer of 1 to 10;
a fluorinated alkylene group represented by general formula:
-(CHF)ₙ₂-CH₂-
wherein n2 is an integer of 1 to 9;
a fluorinated alkylene group represented by:
-(CHF)ₙ₇-CH₂-CH₂-(CH₂)_{q}-
wherein n7 is an integer of 1 to 7, and q is an integer of 1 to 6; or
a fluorinated alkylene group represented by:
-(CHF)ₙ₇-CH₂-CHI-(CH₂)_{q}-
wherein n7 is an integer of 1 to 7, and q is an integer of 1 to 6.

4. The fluorine-containing ether compound according to any one of claims 1 to 3, wherein
R¹-R²-
is
CH₂F-CHF-;
a fluorinated alkyl group represented by general formula:
CH₂F-CHF-(CHF-CHF)ₙ₃-CH₂-
wherein n3 is an integer of 0 to 4; or
a fluorinated alkyl group represented by general formula:
CHF₂-CHF-(CHF-CHF)ₙ₈-CH₂-CHX¹¹-(CH₂)_{q}-
wherein n8 is an integer of 0 to 2, X¹¹ is I or H, and q is an integer of 1 to 6.

5. The fluorine-containing ether compound according to any one of claims 1 to 4, wherein
R³ is single bond; a non-fluorinated alkylene group having 1 to 5 carbon atoms;
a fluorinated alkylene group represented by general formula:
-(CHF)ₙ₄-
wherein n4 is an integer of 1 to 10; or
a fluorinated alkylene group represented by general formula:
-(CHF)ₙ₅-CH₂-
wherein n5 is an integer of 1 to 9.

6. The fluorine-containing ether compound according to any one of claims 1 to 5, wherein
R⁴-R³-
is
a non-fluorinated alkyl group having 1 to 4 carbon atoms; or
a fluorinated alkyl group represented by general formula:
CH₂F-CHF-(CHF-CHF)ₙ₆-CH₂-
wherein n6 is an integer of 0 to 4.

7. The fluorine-containing ether compound according to any one of claims 1 to 6, wherein the fluorine-containing ether compound is at least one selected from the group consisting of CH₂FCHFOCH₃, CH₂FCHFCH₂OCH₃, CH₂FCHFCH₂OCHFCFH₂, CH₂FCHFCH₂OCH₂CH₃, CH₂FCHFCHFCHFCH₂OCH₃, CHF₂-CHF-CH₂-CHI-CH₂-OCH₃, CHF₂-CHF-CH₂-CH₂-CH₂-OCH₃, CHF₂-CHF-CH₂-CH₂-CH₂-OCHFCFH₂, CHF₂-CHF-CH₂-CH₂-CH₂-OCH₂CH₃, CHF₂-CHF-CH₂-CHI-CH₂-CH₂-CH₂-CH₂-OCH₃, and CHF₂-CHF-CH₂-CH₂-CH₂-CH₂-CH₂-CH₂-OCH₃.

8. A composition comprising
the fluorine-containing ether compound according to any one of claims 1 to 7, and
at least one selected from the group consisting of an alcohol, an ether, an alkane, an alkene, a perfluorinated carbon, a perfluorinated tertiary amine, a perfluorinated ether, a cycloalkane, an ester, a ketone, an aromatic compound, a siloxane, a hydrochlorocarbon, a hydrochlofluorocarbon, and a hydrofluorocarbon.

9. A heat-transfer fluid comprising the fluorine-containing ether compound according to any one of claims 1 to 7, or the composition according to claim 8.

10. A solvent for cleaning comprising the fluorine-containing ether compound according to any one of claims 1 to 7, or the composition according to claim 8.
